(19) Europäisches Patentamt
European Patent Office
Office européen des brevets

(11) Publication number: **0 325 289 B1**

(12) **EUROPEAN PATENT SPECIFICATION**

(45) Date of publication of patent specification: **01.09.93**

(51) Int. Cl.5: **D06L 3/02**, C11D 3/395, //C07D209/48,C07D209/66

(21) Application number: **89101003.5**

(22) Date of filing: **20.01.89**

# (54) Bleaching agents comprising imido-aromatic percarboxylic acids.

(30) Priority: **20.01.88 IT 1913188**

(43) Date of publication of application: **26.07.89 Bulletin 89/30**

(45) Publication of the grant of the patent: **01.09.93 Bulletin 93/35**

(84) Designated Contracting States: **AT BE CH DE ES FR GB LI NL SE**

(56) References cited:
**EP-A- 0 170 386**

**J. Chem. Soc., 1962, part III, pages 3821/3822**

(73) Proprietor: **AUSIMONT S.p.A.**
**Foro Buonaparte, 31**
**I-20121 Milano(IT)**

(72) Inventor: **Venturello, Carlo, Dr.**
**135, Via 23 Marzo**
**I-28100 Novara(IT)**
Inventor: **Cavallotti, Caludio**
**3, Via Lorenteggio**
**I-20100 Milan(IT)**
Inventor: **Burzio, Fulvio, Dr.**
**6, Via Francesco Ferrucci**
**I-20100 Milan(IT)**

(74) Representative: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**D-80803 München (DE)**

**EP 0 325 289 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the preparation of solid or liquid detergent compositions containing percarboxylic acids.

Particularly, the present invention relates to the preparation of detergent compositions which contain bleaching agents based on imido-aromatic (poly)percarboxylic acids, which are especially suitable for washing fabrics at low temperatures.

It is known to use peroxygenated bleaching agents, such as the inorganic peroxides (sodium perborate), for washing fabrics. Said bleaching agents, however, are effective only at temperatures above approximately 70 °C and, therefore, they are not suitable for use in low temperature washing processes.

A class of products which show a bleaching action at low temperature are the organic peroxides. Therefore, in the past years, organic percarboxylic acids have found increasing interest in the industrial field, due to energy-saving considerations.

Accordingly, there is a large number of publications concerned with organic peroxyacid compounds endowed with the required properties of sufficient bleaching activity and, in particular, thermal stability, the latter characteristics being essential for an industrial application and a widespread use of such compounds.

Consequently, many organic straight-chain or cylic mono- or di-peracids are known and used, e.g. in detergent compositions.

Examples of already described percarboxylic acids are diperdodecanedioic acid, monoperphthalic acid, diperazelaic acid, substituted diperglutaric and adipic acids, etc.

In particular, formulations based on persalts and/or amide-derived peracids, which are active also at low temperatures, are known (see e.g. EP-A-170 386). These peracids, however, do not belong to the class of the imido-aromatic percarboxylic acids employed according the present invention.

One object of the present invention, therefore, is to provide a process for the preparation of solid or liquid detergent compositions which contain a particular class of (poly)peracids which are particularly effective as bleaching agents for the washing of textile materials.

Another object is to provide a process for the preparation of detergent compositions which contain bleaching agents which can be employed at low temperatures, without damaging the fibers and/or the color of the fabrics.

These, and still other objects which will become even clearer for those skilled in the art from the following description, are achieved, according to the present invention, by a process for the preparation of solid or liquid detergent compositions, which comprises combining at least one imido-aromatic (poly)-percarboxylic acid of the general formula (I):

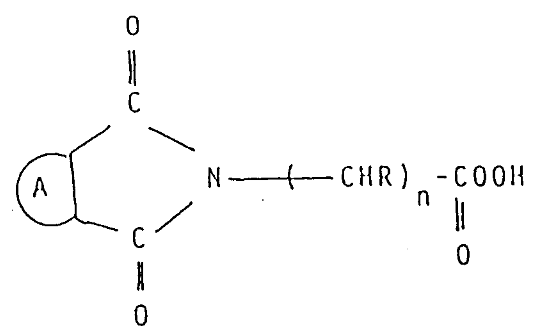

(I)

wherein the groups R, which may be the same or different from each other, represent hydrogen, OH, COOH, COOOH, COOR' or a linear or branched $C_1$-$C_5$ alkyl group which optionally is substituted with one or more groups X selected from $C_1$-$C_5$ alkoxy, OH, COOH, COOOH, COOR', $NO_2$ and halogen; R' is a $C_1$-$C_5$ alkyl group optionally substituted with one or more groups X as defined above; A represents a benzene or naphthalene ring which optionally is substituted with one or more groups selected from $C_1$-$C_5$ alkyl groups and groups X as defined above; and n is an integer of from 1 to 5;
with at least one other component and/or additive selected from builders, surfactants, soaps, zeolites, hydrotropic agents, corrosion inhibitors, enzymes, optical bleaching agents, stabilizers and other peroxy compounds.

The above alkyl groups R have 1 to 5 carbon atoms. Examples of $C_{1-5}$ alkyl groups are methyl, ethyl, n- and i-propyl, n-, i-, sek- and ter.-butyl and pentyl. Said groups may optionally be substituted, e.g. with one or more (preferably 1 to 3) radicals selected from $C_{1-5}$ alkoxy (e.g. methoxy and ethoxy), OH, COOH

or COOOH, COOR', $NO_2$ and halogen (e.g. F, Cl and Br). Examples of such groups are $-CH_2OCH_3$,-$CH_2OC_2H_5$,$-CH_2OH$, $-CH_2COOH$, $-CH_2COOOH$, $-CH_2CH_2COOH$,$-CH_2CH_2COOOH$, $CH_2Cl$, $-CH_2F$, $CF_3$ and $-CH_2COOC_2H_5$.

Likewise, the group A may carry one or more (up to 4 and preferably 1 or 2) substituents. Said substituents are selected . from the ones recited above as substituents for the alkyl groups R and, additionally, from $C_{1-5}$ alkyl groups. Particularly preferred substituents are COOH and COOOH radicals.

Substituents for the $C_{1-5}$ alkyl groups R' are the same as those already mentioned above in connection with the groups R.

A particularly preferred meaning of R is hydrogen.

The following imido-aromatic (poly)peracids of formula (I) have been shown to be particularly effective in the above detergent compositions: phthalimido-peracetic acid, 3-phthalimido-perpropionic acid, 4-phthalimido-perbutyric acid, 2-phthalimido-di-perglutaric acid, 2-phthalimido-di-persuccinic acid, 3-phthalimido-perbutyric acid, 2-phthalimido-per-propionic acid, methyl half-ester of 2-phthalimido-mono-perglutaric acid, 3-phthalimido-di-peradipic acid, naphthalimido-peracetic acid, 2-phthalimido-mono-persuccinic acid, 4-(4-percarboxy)-phthalimido-per-butyric acid.

Said acids may be obtained according to substantially conventional methods. For example, they may be prepared by the reaction of the imido-aromatic (poly)carboxylic acid of a structure corresponding to the desired peracid of formula (I) with $H_2O_2$ in sulphuric or methanesulphonic acid and by subsequent separation and so forth, according to known techniques, or by operating in an alkaline medium, always according to known methods, starting from the corresponding anhydrides.

When at least one -(CHR)- residue present in the formula of the starting substrate comprises a carboxylic group it is possible to prepare the corresponding peracid of formula (I) by using the corresponding anhydride.

In this case, depending on the reaction conditions (acidic or alkaline medium and so forth), di- or mono-peracids, i.e. compounds containing two percarboxylic acid groups or one percarboxylic acid group and one carboxylic acid group may selectively be obtained.

According to a preferred procedure, the percarboxylation reaction of the acid or poly-acid used as the starting substrate, is carried out by gradually adding $H_2O_2$, having a concentration of from about 70% to about 90% by weight, to a solution of the acid in concentrated $H_2SO_4$, or in $CH_3SO_3H$, maintaining the reaction temperature throughout the reaction within the range of from about 15° to about 50°C, depending on the reactivity of the substrate.

The amount of $H_2SO_4$ or $CH_3SO_3H$, determined at a concentration of 100%, preferably is from 3 to 20 moles per mole of substrate, particularly from about 4 to 14 moles per mole of substrate.

The hydrogen peroxide preferably is used in amounts in excess of that of the substrate. Generally the amounts range from about 2 to 6 moles $H_2O_2$ per mole of substrate, particularly from about 2.2 to 5 moles per mole of substrate, depending on the COOH groups to be percarboxylated.

The reaction time depends on the nature of the substrate, on the reaction temperature, and the total $H_2SO_4/H_2O$ or $CH_3SO_3H/H_2O$ molar ratio at the end of the reaction. Said ratio preferably ranges from about 1 to 6 and, particularly, from about 1.6 to 4, by choosing suitable values for the various parameters involved.

Reaction times of from about 30 minutes to 2 hours are generally required.

The separation of the imido-aromatic (poly)percarboxylic acid of formula (I) may be carried out according to conventional techniques, such as e.g. filtration of the solid precipitate obtained after treatment of the reaction mixture with an ammonium sulfate solution, solvent extraction etc.

The imido-aromatic (poly)percarboxylic acids of formula I may thus be obtained in the form of crystalline solids.

The substrates, used as the starting materials, are per se known compounds, or can be prepared according to conventional methods. Examples of suitable substrates are phthalimido-acetic acid, 3-phthalimido-propionic acid, 4-phthalimido-butyric acid, 2-phthalimido-glutaric acid and the corresponding anhydride, 2-phthalimido-succinic acid and the corresponding anhydride, 3-phthalimido-butyric acid, 2-phthalimido-propionic acid, methyl half-ester of 2-phthalimido-glutaric acid, 3-phthalimido-adipic acid, naphthalimido-acetic acid, phthaloyl serine, 4-(4-carboxy)-pthalimido-butyric acid, and so forth, from which the above preferred peracids of formula (I) are obtained.

The percarboxylic acid products of formula (I) are usually solid at room temperature.

According to the present invention, they are used for the preparation of detergent formulations, e.g. granular formulations, as bleaching agents in solution over a wide temperature range, e.g. of from 20°C to 90°C.

Therefore, the imido-aromatic peracids of formula (I) are used as bleaching agents, associated to and incorporated into conventional detergent compositions which are used within the above defined temperature range and contain other components and/or additives, selected from builders, surfactants, soaps, zeolites, hydrotropic agents, corrosion inhibitors, enzymes, optical brighteners, stabilizers and other peroxy compounds.

Preferably, the working temperature ranges from room temperature to about 65 °C.

The preparation and use of the compositions and the corresponding formulations are carried out according to the described and/or conventional techniques.

The imido-aromatic peracids of formula (I) are used in solid and liquid detergent compositions in the presence or absence of other bleaching (peroxy) compounds.

Further, the above imido-aromatic peracids may be subjected to a known phlegmatization process.

The following examples serve to illustrate the present invention without being a limitation of the scope thereof.

The products prepared in the examples were characterized by elemental analysis, determination of their content of active oxygen (by iodometric titration), and by Fourier Transform Infrared Spectroscopy (FT-IR).

In the following the term "EO" means ethylene oxide; the percentages are expressed by weight. "DIXAN" and "BIOPRESTO" are trade marks of commercially available detergents produced by HENKEL - ITALY and LEVER - ITALY, respectively.

## Example 1

330 g (3.434 mole) of methanesulphonic acid were placed in a beaker equipped with stirrer, thermometer and external bath.

The internal temperature was increased to 25 °C and 55 g (0.268 mole) of phthalimido-acetic acid were added under stirring within 15 minutes.

The temperature was then lowered to 10 °C and 44 g of $H_2O_2$ (70%, 0.906 mole) were gradually added under stirring, at a rate such as to maintain the temperature below 15 °C.

Stirring was continued at 15 °C for 1.5 hours.

At the end, the reaction mixture was poured into 600 ml of 20% $(NH_4)_2SO_4$, maintained under stirring at 5 °C.

Stirring was continued for 15 minutes at a temperature between 5 °-10 °C.

The solid product was filtered under vacuum over a porous septum. The thus obtained product was suspended in 400 ml of 8% $Na_2SO_4$ and neutralized (pH 6) by addition of a 15% $Na_2CO_3$ solution.

The resulting solid was then filtered, washed with ice water (100 ml), wiped and dried on a porous plate in a $CaCl_2$-drier under vacuum (2 mm Hg) at room temperature.

There were obtained 58 g of substantially pure phthalimido-peracetic acid. Yield: 97%.

The product may be recrystallized by dissolving it in ethyl acetate and by adding petroleum ether until the solution becomes turpid.

## Elemental Analysis:

Calculated for $C_{10}H_7O_5N$: C: 54.30%; H: 3.19%; N: 6.33%; O (active): 7.23%.
Found: C: 54.32%; H: 3.33%; N: 6.57%; O (active): 7.2%.
Melting Point: 118 °C (with decomposition).

## Example 2

28 g (0.274 mole) of 96% $H_2SO_4$ were introduced into a beaker equipped with stirrer, thermometer and external bath.

The inside temperature was brought to 25 °C and 11.7 g (0.0534 mole) of 3-phthalimido-propionic acid were added under stirring within 15 minutes.

The temperature was lowered to 10 °C and 5.2 g $H_2O_2$ (70%, 0.107 mole) were gradually added under stirring, at a rate such as to maintain the temperature below 15 °C.

The stirring was continued at 15 °C for 1.5 hours. At the end, the reaction mixture was poured into 80 ml of 20% $(NH_4)_2SO_4$, maintained under stirring at 5 °C. Stirring was continued for 15 minutes at a temperature between 5 and 10 °C.

The solid product was filtered under vacuum over a porous septum.

The thus obtained product was suspended in 50 ml of 8% $Na_2SO_4$ and neutralized by adding (pH 6) 15% $Na_2CO_3$. The resulting solid was again filtered, washed with 20 ml of ice water, wiped and dried on a porous plate in a $CaCl_2$-drier under vacuum (0.27 kPa = 2 mm Hg) at room temperature.

11.3 g of substantially pure 3-phthalimido-perpropionic acid were thus obtained. Yield: 90%.

The product may be recrystallized as described in Example 1.

Elemental Analysis:

Calculated for $C_{11}H_9O_5N$: C: 56.17%; H: 3.85%; N: 5.95%; O (active): 6.80%.
Found: C: 56.83%; H: 4.01%; N: 6.10%; O (active): 6.79%.
Melting point: 91 °C (with decomposition).

Example 3

The procedure of Example 2 was repeated, replacing 3-phthalimido-propionic acid by 4-phthalimido-butyric acid (15 g; 0.0643 mole) and using 30 g of 96% $H_2SO_4$ (0.294 mole) and 7 g of $H_2O_2$ (70%, 0.144 mole) and prolonging the reaction time to 2 hours.

14.5 g of substantially pure 4-phthalimido-perbutyric acid were obtained. Yield: 90%.

The product may be recrystallized as described in Example 1.

Elemental Analysis:

Calculated for $C_{12}H_{11}O_5N$: C: 57.83%; H: 4.45%; N: 5.62%; O (active): 6.42%.
Found: C: 57.98%; H: 4.52%; N: 5.69%; O (active): 6.41%.
Melting point: 103 °C (with decomposition).

Example 4

The procedure of Example 1 was repeated, replacing phthalimido-acetic acid by 2-phthalimido-glutaric acid (6 g; 0.0216 mole) and using 28 g (0.291 mole) of methanesulphonic acid and 3.5 g of $H_2O_2$ (85%, 0.0875 mole).

At the end, 15 ml of 40% $(NH_4)_2SO_4$ were gradually added to the reaction mixture, cooled to 0 °C, at a rate such that the temperature was maintained between 0 and 5 °C.

The resulting mixture was extracted with $Et_2O$ (6 x 30 ml).

The ether extract was washed with 30 ml of 40% $(NH_4)_2SO_4$, dried over anhydrous $Na_2SO_4$, filtered and evaporated.

An oil was obtained which was dissolved in $Et_2O$ (20 ml) and precipitated, as a solid, by adding petroleum ether (40 ml) and maintaining the mixture under agitation up to complete solidification.

After filtration, 5.8 g of 2-phthalimido-diperglutaric acid (95%) were obtained. Yield: 82%.

The product was recrystallized as described in Example 1.

Elemental Analysis:

Calculated for $C_{13}H_{11}O_8N$: C: 50.49%; H: 3.58%; N: 4.53%; O (active): 10.34%.
Found: C: 49.96%; H: 3.75%; N: 4.70%; O (active): 10.33%.
Melting point: 112 °C (with decomposition).

Example 5

The procedure of Example 4 was repeated, replacing 2-phthalimido-glutaric acid by 2-phthalimido-succinic acid (5 g; 0.019 mole), using 20 g (0.208 mole) of methanesulphonic acid, 3.8 g (0.095 mole) of 85% $H_2O_2$ and extending the reaction time to 2 hours.

At the end, 80 ml of a 40% $(NH_4)_2SO_4$ solution were gradually added to the reaction mixture, cooled to 0 °C, at a rate such that the temperature was maintained between 0 and 5 °C.

Stirring was continued for 15 minutes at 0-5 °C.

The procedure described in Example 2 was then followed.

4 g of substantially pure 2-phthalimido-dipersuccinic acid were obtained. Yield: 71%.

The product may be recrystallized as described in Example 1.

Elemental Analysis:

Calculated for $C_{12}H_9O_8N$: C: 48.82%; H: 3.07%; N: 4.74%; O (active): 10.84%.
Found: C: 48.44%; H: 3.22%; N: 4.88%; O (active): 10.82%.
Melting point: 131 °C (with decomposition).

Example 6

The procedure of Example 5 was repeated, replacing 2-phthalimido-succinic acid by 2-phthalimido-succinic anhydride (2 g; 0.0082 mole), using 10 g (0.104 mole) of methanesulphonic acid and 1.3 g (0.0325 mole) of 85% $H_2O_2$, and reducing the reaction time to 1.5 hours.

At the end, 60 ml of a 20% $(NH_4)_2SO_4$ solution were gradually added to the reaction mixture, cooled to 0 °C, at a rate such that the temperature was maintained between 0 and 5 °C.

The resulting mixture was extracted with EtOAc/$Et_2O$ 1:2 (2 x 30 ml). The organic extract was washed with 20 ml of a 20% $(NH_4)_2SO_4$ solution, dried over anhydrous $Na_2SO_4$, filtered and evaporated under vacuum.

1.8 g of 2-phthalimido-dipersuccinic acid (95%) were obtained. O (active) found: 10.3%; O (active) calculated for $C_{12}H_9O_8N$: 10.84%.

Example 7

5 g of a 17.4% $Na_2CO_3$ solution were placed in a 50 ml beaker. The internal temperature was brought to 5 °C and 0.8 g of 85% $H_2O_2$ and 0.04 g of $MgSO_4 . 7 H_2O$ were introduced.

While maintaining a temperature of 5 °C, 2 g of 2-phthalimido-succinic anhydride (0.0082 mole) were successively and rapidly added.

The internal temperature was allowed to gradually increase to 20 °C by continuing stirring for 30 minutes.

30 ml of ethyl ether and 4.2 g of 20% $H_2SO_4$ were then added. The ether layer was successively separated, washed with a 40% $(NH_4)_2SO_4$ solution (2 x 20 ml) and dried over anhydrous $Na_2SO_4$. Then, after the filtration of the sulphate, the peracid was precipitated by adding 30 ml of petroleum ether and stirring the mixture at room temperature for 30 minutes. The peracid was filtered and dried under vacuum at room temperature.

1.5 g of 2-phthalimido-mono-persuccinic acid (63%) were obtained.
O (active) found: 3.6%; O (active) calculated for $C_{12}H_9NO_7$: 5.73%.

Example 8

1.5 g of 85% $H_2O_2$ (0.0375 mole) were added, under stirring at 15-20 °C, to 2 g of a suspension of 4-(4-carboxy)-phthalimido-butyric acid (0.0072 mole) in 12 g (0.125 mole) of methanesulphonic acid.

Stirring was continued for 2 hours at 15 °C.

The reaction product was then poured in 40 ml of a 40% $(NH_4)_2SO_4$ solution maintained at 5 °C and, after 15 minutes of stirring, the separated solid product was filtered. The solid was then neutralized (pH 6) by suspending it in a 8% $Na_2SO_4$ solution and by adding a 15% $Na_2CO_3$ solution.

The resulting solid was again filtered, washed with ice water (30 ml) and dried on a porous plate in a $CaCl_2$ drier.

The product may be recrystallized by dissolving it in ethyl acetate at room temperature and precipitation thereof by adding petroleum ether.

There were thus obtained 2 g of substantially pure 4-(4-percarboxy)-phthalimido-perbutyric acid. Yield: 90%.

Elemental Analysis:

Calculated for $C_{13}H_{11}O_8N$: C: 50.49%; H: 3.58%; N: 4.53%; O (active): 10.35%.
Found: C: 50.04%; H: 3.75%; N: 4.48%; O (active): 10.34%.
Melting point: 109 °C (with decomposition).

Example 9 (Application example)

Bleaching with phthalimido-peracetic acid (FIPA)

Bleaching tests were carried out with a detergent formulation containing FIPA (composition D) in the amounts given in the following Table 1, in comparison to similar compositions containing, as bleaching agents, tetrahydrated sodium perborate (PBS) (composition A), PBS activated with TAED (tetraacetylethylenediamine) in stoichiometric amounts (composition B), and H 48 peracid (Mg salt of mono-perphthalic acid), produced by INTEROX Chemical Ltd. London; G.B., (composition C). Two of the best detergents known in Italy, i.e. BIO PRESTO$^R$ (composition E) and DIXAN$^R$ (composition F) were also included in the test as comparison.

BIO PRESTO$^R$ contains TAED as activator and, therefore, is suitable for washing at low temperatures and DIXAN$^R$, a detergent without activator but with high concentrations of PBS, is suitable for washing at high temperatures.

The not commercially available formulations were prepared by dry blending of a detergent base, common to all the above formulations and defined hereinafter, with the listed bleaching products. As detergent base a granular composition was used which contained all the normal (conventional) components of a detergent for a washing machine (surfactants, builders, etc.), except the chemical bleaching agents, obtained by atomization of a mixture of the above components.

The detergent base used had the following composition:

| | Weight % |
|---|---|
| Total surfactants (Sodium alkyl ($C_{12}$)benzenesulphonate, soap, alcohol ($C_{16}$-$C_{18}$) ethoxylate 7 EO) | 15.4 |
| Total sodium phosphates | 8.8 |
| Zeolite A | 19.8 |
| Silicate ($SiO_2/Na_2O = 2$) | 4.4 |
| Sodium sulphate | 36.6 |
| Sodium carbonate | 6.6 |
| Carboxymethylcellulose | 1.1 |
| Anti-encrusting copolymers | 4.8 |
| Water | 2.2 |
| Optical bleaching agents | 0.3 |

The tested bleaching agents were employed in amounts such that each formulation had the same content of active oxygen (1.4%). Where necessary, sodium sulphate was added to complete the composition.

The commercial detergents used as comparison had the following composition:

| | BIO PRESTO$^R$ Weight % | DIXAN$^R$ Weight % |
|---|---|---|
| Total surfactants (Straight alkyl ($C_{12}$)benzenesulphonate, soap, alcohol ($C_{16}$-$C_{18}$) ethoxylate 7 EO) | 13.8 | 12.7 |
| Total sodium phosphates | 6.6 | 8.0 |
| Zeolite A | 18.4 | 16.6 |
| Sodium silicate ($SiO_2/Na_2O = 2$) | 3.9 | 2.8 |
| Monohydrated sodium perborate | 8.3 | - |
| Tetrahydrated sodium perborate | - | 27.6 |
| Sodium sulphate | 33.2 | 22.0 |
| Sodium carbonate | 6.2 | 10.6 |
| Carboxymethylcellulose | 1.0 | 1.0 |
| Anti-encrusting copolymers | 4.5 | 3.5 |
| Tetraacetylethylenediamine (activator) | 1.8 | - |
| Water | 1.9 | 0.4 |
| Optical bleaching agents, enzymes, perfume and others | to 100 | to 100 |

The tested formulations (A - F) had the compositions summaized in the following Table 1.

TABLE 1

| | |
|---|---|
| Composition A | |
| Detergent base | 80 % |
| PBS (10% active oxygen) | 14 % |
| $Na_2SO_4$ | 6 % |
| Composition B | |
| Detergent base | 75 % |
| PBS (10% of active oxygen) | 14 % |
| TAED (95% of active substance) | 11 % |
| Composition C | |
| Detergent base | 74 % |
| H.48 (5.5% of active oxygen) | 26 % |

TABLE 1 (continued)

| | |
|---|---|
| Composition D | |
| Detergent base | 80 % |
| FIPA (7.1% of active oxygen) | 20 % |
| Composition E | |
| BIO PRESTO[R] detergent for washing machines of LEVER containing 13% of PBS ( in the form of tetrahydrate) + about 2% of TAED | |
| Composition F | |
| DIXAN[R] commercial detergent for washing machines of HENKEL containing approximately 28% of tetrahydrated PBS | |

The tests were carried out in an IGNIS MOD.644 commercial washing machine, introducing into the machine two cotton specimens (15 x 15 cm) stained in standard manner with red wine at the EMPA INSTITUTE of St.Gallen (Switzerland) and marked with the "EMPA 114" mark, together with 3 kg clean cotton dusters, as ballast, for each washing cycle.

The dosage was 150 g in each washing cycle for each formulation.

The washing processes were carried out by standard programs at medium temperature (about 60 °C) and at low temperature (about 40 °C). A program at high temperature (85-90 °C) was used only for DIXAN[R] in order to determine the maximum level of bleaching which may actually be reached. Normal, undistilled tap water, having a hardness of 14 °F, was used.

The results of the tests are shown in the following Table 2, wherein the data are expressed as % bleaching, determined by the following equation:

$$\% \text{ Bleaching} = \frac{A - B}{C - B} \times 100$$

wherein:

A = degree of whiteness (%) of the specimen bleached after the test;

B = degree of whiteness (%) of the specimen before the test;

C = degree of whiteness (%) of the completely bleached specimen.

The degree of whiteness was measured by means of an Elrepho Zeiss Reflectometer using a filter N.6 ($\lambda$ = 464 nm) and assuming MgO = 100% of whiteness.

TABLE 2

| % Bleaching | | | |
|---|---|---|---|
| | **Washing Program** | | |
| | At low temperature (40 °C) | At medium temperature (60 °C) | At high temperature (85-90 °C) |
| Composition A | 33.3 | 43.7 | ... |
| Composition B | 51.4 | 74.8 | ... |
| Composition C | 63 | 76 | ... |
| Composition D | 79.4 | 89.9 | ... |
| Composition E | 32.8 | 66.1 | |
| Composition F | ... | --- | 92.5 |

The data show that:

- the FIPA bleaching power exceeds that of all the other tested bleaching formulations; at medium temperature it allows to obtain bleaching results very close to the maximum ones, which may be obtained only at high temperature and by using high amounts of active oxygen (DIXAN);
- excellent results, much better than those of the other formulations, may be obtained - ever more surprisingly - by FIPA at low temperature;
- the activated PBS is less effective than the peracids at low temperature even if the activation is complete (see B v. C and D).

The modest activator concentration in the commercial detersive (E), due mostly to storage stability problems, leads to the result that the bleaching activity at low temperature cannot be differentiated from that of non-activated PBS (A) and that this activity at medium temperature is very far from that which may potentially be obtained by activator (B).

Examples 10 - 13 (Application examples)

Bleaching tests were carried out with the same concentrations of active oxygen in the bleaching solution, and by using the imido-aromatic peracids of formula (I), shown in the following Table 3, in comparison to H.48.

All tests were carried out at a constant temperature of 60 °C, with an initial concentration of total active oxygen in the bleach (equal for all products) of 200 mg/l.

Procedure

For each test, 500 ml of deionized water, contained in a 1,000 ml flask equipped with a condenser, were heated to a temperature of 60 °C and adjusted to a pH value of 9.5 (with a few drops of NaOH solution). Then, the bleaching product was added, under stirring, in the amounts given in the following Table 3, and immediately thereafter, two cotton specimens (10 x 10 cm) stained in standard manner by red wine at the EMPA INSTITUTE of St.Gallen (Switzerland), and marked with the "EMPA 114" mark, were added.

The system was subsequently stirred for 60 minutes and, at the end of this time, the specimens, rinsed under running water, were dried and ironed, and were then subjected to the evaluation of the bleaching effect by determining the degree of whiteness by reflectometry. The results are reported in the following Table 3, expressed as % Bleaching as defined in the above example 9.

The data in Table 3 show that the peracids of formula (I) have a bleaching power comparable to that of H.48 and in some cases even higher.

Table 3

| COMPOUND | Amounts used in the test (grams) | Initial concentration of total active oxygen (mg/l) | % Bleaching |
|---|---|---|---|
| - Example 1 (titer: 7.2% of active oxygen) | 1.46 | 200 | 83.6 |
| - Example 2 (titer: 6.79% of active oxygen) | 1.47 | 200 | 83.0 |
| - Example 3 (titer: 6.41% of active oxygen) | 1.56 | 200 | 79.4 |
| - Example 4 (titer: 9.81% of active oxygen) | 1.02 | 200 | 74.0 |
| - Example 5 (titer: 10.82% of active oxygen) | 0.924 | 200 | 75.0 |
| - H 48 (titer: 5.5% of active oxygen) | 1.86 | 200 | 75.1 |

## Claims

1. Process for the preparation of solid or liquid detergent compositions, which comprises combining at least one imido-aromatic (poly)percarboxylic acid of the general formula (I):

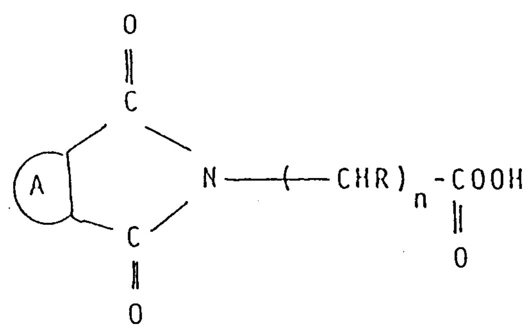

(I)

wherein the groups R, which may be the same or different from each other, represent hydrogen, OH, COOH, COOOH, COOR' or a linear or branched $C_1$-$C_5$ alkyl group which optionally is substituted with one or more groups X selected from $C_1$-$C_5$ alkoxy, OH, COOH, COOOH, COOR', $NO_2$ and halogen; R' is a $C_1$-$C_5$ alkyl group optionally substituted with one or more groups X as defined above; A represents a benzene or naphthalene ring which optionally is substituted with one or more groups selected from $C_1$-$C_5$ alkyl groups and groups X as defined above; and n is an integer of from 1 to 5;
with at least one other component and/or additive selected from builders, surfactants, soaps, zeolites, hydrotropic agents, corrosion inhibitors, enzymes, optical bleaching agents, stabilizers and other peroxy compounds.

2. Process according to claim 1, wherein the group(s) R are selected from linear or branched $C_1$-$C_5$ alkyl groups, optionally substituted by at least one $C_1$-$C_5$ alkoxy, $NO_2$, or OH radical.

3. Process according to claim 1, wherein R is hydrogen.

4. Process according to anyone of claims 1 to 3, wherein A is an unsubstituted benzene ring.

5. Process according to anyone of claims 1 to 3, wherein said at least one imido-aromatic (poly)-percarboxylic acid is selected from phthalimido-peracetic acid, 3-phthalimido-perpropionic acid, 4-phthalimido-perbutyric acid, 2-phthalimido-diperglutaric acid, 2-phthalimido-dipersuccinic acid, 3-phthalimido-perbutyric acid, 2-phthalimido-perpropionic acid, methyl half-ester of 2-phthalimido-monoperglutaric acid, 3-phthalimido-diperadipic acid, naphthalimido-peracetic acid, 2-phthalimido-monopersuccinic acid and 4-(4-percarboxy)phthalimido-perbutyric acid.

6. Use of the detergent compositions obtainable according to the process of any one of claims 1 to 5 at a temperature of from room temperature to 65 °C.

**Patentansprüche**

1. Verfahren zur Herstellung fester oder flüssiger Detergent-Zusammensetzungen, welches die Kombination umfaßt von: wenigstens einer imidoaromatischen (Poly)percarbonsäure der allgemeinen Formel (I):

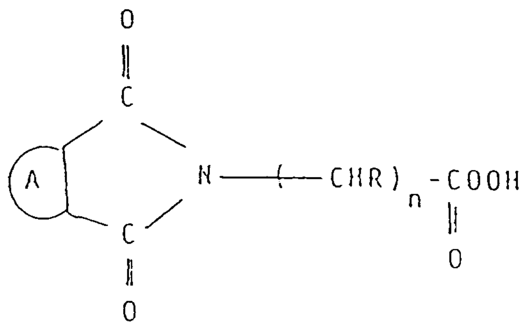

(I)

worin die Gruppen R, die gleich oder verschieden sein können, Wasserstoff, OH, COOH, COOOH, COOR' oder eine lineare oder verzweigtkettige $C_1$-$C_5$-Alkylgruppe, gegebenenfalls substituiert mit einer oderer mehreren Gruppen X, ausgewählt aus $C_1$-$C_5$-Alkoxy, OH, COOH, COOOH, COOR', $NO_2$ und Halogen, bedeuten; R' eine $C_1$-$C_5$-Alkylgruppe, gegebenenfalls substituiert mit einer oder mehreren Gruppen X - wie oben definiert - bedeutet; A einen Benzol- oder Naphthalinring, gegebenenfalls substituiert mit einer oder mehreren Gruppen, die ausgewählt werden aus $C_1$-$C_5$Alkylgruppen und Gruppen X - wie oben definiert - bedeutet; und n eine ganze Zahl von 1 bis 5 ist;
mit wenigstens einer anderen Komponente und/oder einem anderen Additiv, ausgewählt aus Aufbaustoffen, oberflächenaktiven Mitteln, Seifen, Zeolithen, hydrotropen Mitteln, Korrosionshemmern, Enzymen, optischen Bleichmitteln, Stabilisatoren und anderen Peroxyverbindungen.

2. Verfahren nach Anspruch 1, worin die Gruppe(n) R ausgewählt werden aus linearen oder verzweigtkettigen $C_1$-$C_5$-Alkylgruppen, gegebenenfalls substituiert mit wenigstens einer $C_1$-$C_5$-Alkoxy-, $NO_2$- oder OH-Gruppe.

3. Verfahren nach Anspruch 1, worin R Wasserstoff ist.

4. Verfahren nach einem der Ansprüche 1 bis 3, worin A ein nicht-substituierter Benzolring ist.

5. Verfahren nach einem der Ansprüch 1 bis 3, worin die genannte wenigstens eine imidoaromatische (Poly)percarbonsäure ausgewählt wird aus Phthalimido-peressigsäure, 3-Phthalimido-perpropionsäure, 4-Phthalimido-perbuttersäure. 2-Phthalimido-diperglutarsäure, 2-Phthalimido-diperbernsteinsäure, 3-Phthalimido-perbuttersäure, 2-Phthalimidoperpropionsäure, Methyl-Halbester von 2-Phthalimidomonoperglutarsäure, 3-Phthalimido-diperadipinsäure, Naphthalimido-peressigsäure, 2-Phthalimido-monoperbernsteinsäure und 4-(4-Percarboxy)phthalimido-perbuttersäure.

6. Verwendung der Detergent-Zusammensetzungen, erhältlich nach dem Verfahren nach einem der Ansprüche 1 bis 5, bei einer Temperatur zwischen Raumtemperatur und 65° C.

**Revendications**

1. Procédé pour la préparation de compositions détergentes liquides ou solides qui comprend la combinaison d'au moins un acide poly(percarboxylique)imidoaromatique de formule générale (I):

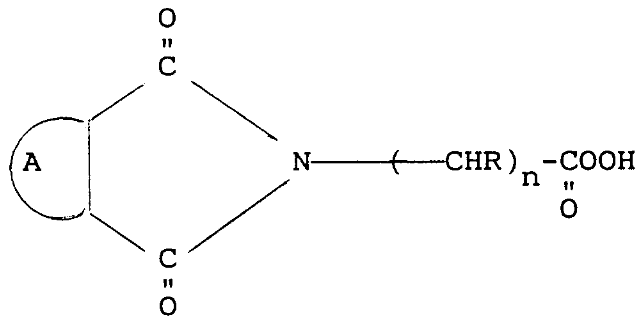

(I)

dans laquelle:

les groupes R, qui peuvent être identiques ou différents les uns des autres, représentent un atome d'hydrogène, ou groupe OH, COOH, COOOH, COOR' ou un radical alkyle en $C_1$ à $C_5$, linéaire ou ramifié, qui est éventuellement substitué par un ou plusieurs groupes X sélectionnés dans les groupes des restes alcoxy en $C_1$ à $C_5$, OH, COOH, COOOH, COOR', $NO_2$ et halogène; R' étant un groupe alkyle en $C_1$ à $C_5$, éventuellement substitué par un ou plusieurs groupes X, tels que définis ci-dessus;

A représente un cycle benzène ou naphtalène qui est éventuellement substitué par un ou plusieurs groupes sélectionnés dans le groupe des radicaux alkyles en $C_1$ à $C_5$, et les groupes X tels que définis ci-dessus; et

n est un nombre entier compris entre 1 et 5; avec au moins un autre composant et/ou additif sélectionné dans les adjuvants, les tensioactifs, les savons, les zéolites, les agents hydrotropes, les inhibiteurs de corrosion, les enzymes, les agents de blanchiment, les stabiliseurs et les autres composés peroxy.

**2.** Procédé selon la revendication 1, dans lequel les groupes R sont sélectionnés dans le groupe des radicaux alkyles en $C_1$ à $C_5$, linéaires ou ramifiés, éventuellement substitués par au moins un groupe alcoxy en $C_1$ à $C_5$, $NO_2$ ou OH.

**3.** Procédé selon la revendication 1, dans lequel R est un atome d'hydrogène.

**4.** Procédé selon l'une quelconque des revendications 1 à 3, dans lequel A est un cycle benzène non-substitué.

**5.** Procédé selon l'une quelconque des revendications 1 à 3, dans lequel au moins un desdits acides (poly)percarboxyliques imidoaromatiques est sélectionné dans le groupe comprenant l'acide phtalimidoperacétique, l'acide 3-phtalimidoperpropionique, l'acide 4-phtalimidoperbutyrique, l'acide 2-phtalimidodiperglutarique, l'acide 2-phtalimidodipersuccinique, l'acide 3-phtalimidoperbutyrique, l'acide 2-phtalimidoperpropionique, l'ester demi méthylé d'acide 2-phtalimidomonoperglutarique, l'acide 3-phtalimidodiperadipique, l'acide naphtalimidoperacétique, l'acide 2-phtalimidomonopersuccinique et l'acide 4-(4-percarboxy)-phtalimidoperbutyrique.

**6.** Utilisation de compositions de détergents obtenus selon le procédé suivant l'une quelconque des revendications 1 à 5, à une température comprise entre la température ambiante à 65 °C.